# EUROPEAN PATENT SPECIFICATION

(11) **EP 2 126 100 B1**
(45) Date of publication and mention of the grant of the patent: **14.11.2012**
(21) Application number: 08735442.9
(22) Date of filing: 20.03.2008
(51) Int. Cl.: C12N 15/90

(54) **IMPROVED METHOD FOR HOMOLOGOUS RECOMBINATION**
VERBESSERTES VERFAHREN ZUR HOMOLOGEN REKOMBINATION
PROCÉDÉ AMÉLIORÉ DE RECOMBINAISON ALLOGÉNIQUE

(30) Priority: 21.03.2007 EP 07104588; 05.07.2007 EP 07111826
(43) Date of publication of application: 02.12.2009
(60) Divisional application: 12182937.8
(73) Proprietor: DSM IP Assets B.V., 6411 TE Heerlen (NL)
(72) Inventor: TOUW-RIEL, Hesselien, NL-2645 GC Delfgauw (NL); BERG, VAN DEN, Marco Alexander, NL-2685 EH Poeldijk (NL)
(74) Representative: De Vroom, Erik
(86) International application number: PCT/EP2008/053352
(87) International publication number: WO 2008/113847

(56) References cited:
- WO-A-2005/095624
- WO-A-2007/013979
- NIELSEN ET AL: "Efficient PCR-based gene targeting with a recyclable marker for Aspergillus nidulans" FUNGAL GENETICS AND BIOLOGY, SAN DIEGO, CA, US, vol. 43, no. 1, January 2006 (2006-01), pages 54-64, XP005234491 ISSN: 1087-1845
- NINOMIYA Y ET AL: "Highly efficient gene replacements in Neurospora strains deficient for nonhomologous end-joining" PROCEEDINGS OF THE NATIONAL ACADEMY OF SCIENCES OF USA, NATIONAL ACADEMY OF SCIENCE, WASHINGTON, DC, US, vol. 101, no. 33, 17 August 2004 (2004-08-17), pages 12248-12253, XP002346025 ISSN: 0027-8424

## Description

### Field of the invention

The present invention relates to an improved method for efficient and targeted integration of nucleic acids into chromosomes of cells.

### Detailed description of the invention

Different cell types are used for different industrial purposes. For example mammalian cell lines are used for antibody production; fungal cells are preferred organisms for production of polypeptides and secondary metabolites; bacterial cells are preferred for small metabolite and antibiotic production; plant cells are preferred for taste and flavor compounds. Recombinant techniques are widely employed for optimization of the productivity of cells and/or processes. This can involve a multitude of options, including, but not limited to over expression of a gene of interest, deletion or inactivation of competing pathways, changing compartmentalization of enzymes, increasing protein or metabolite secretion, increasing organelle content and the like (see for example Khetan and Hu (1999) In: Manual of Industrial Microbiology Biotechnology, Eds. Demain and Davies, pg. 717-724). To be successful with these methods it is crucial that the recombinant construct is stably maintained in the production host. This can be either as part of an episomal vector or via integration in the genome. The latter situation is the preferred solution as this is the most stable situation. Even more preferred is the integration at the predetermined, correct genomic locus. Since in several species, especially most eukaryotic organisms, integration of DNA into the genome occurs with high frequency at random, the construction of industrial production cells by recombinant DNA technology often leads to the unwanted integration of the polynucleotide resulting in genome modifications at random. Moreover, this often results in multiple integrations and thus instable situations. This uncontrolled "at random multiple integration" of a polynucleotide is a potentially dangerous process, which can lead to unwanted modification of the genome of the host, resulting in decreased productivity.

It is therefore highly desirable to be able to construct an industrial production cell line by correct genome targeting of the polynucleotide of interest with high efficiency. Furthermore, now that the sequences of complete genomes of an increasing amount of organisms are becoming available, the opportunity to construct genome-wide over expression and deletion libraries is opened. Important requirements for the efficient construction of such libraries are that the organism in question can be efficiently transformed, that the polynucleotide of interest is correctly targeted with a high frequency and that the required homology needed to direct targeted integration of a nucleic acid into the genome is relatively short.

There are several methods described to decrease the frequency of this unwanted, at random integration of polynucleotides in cells.

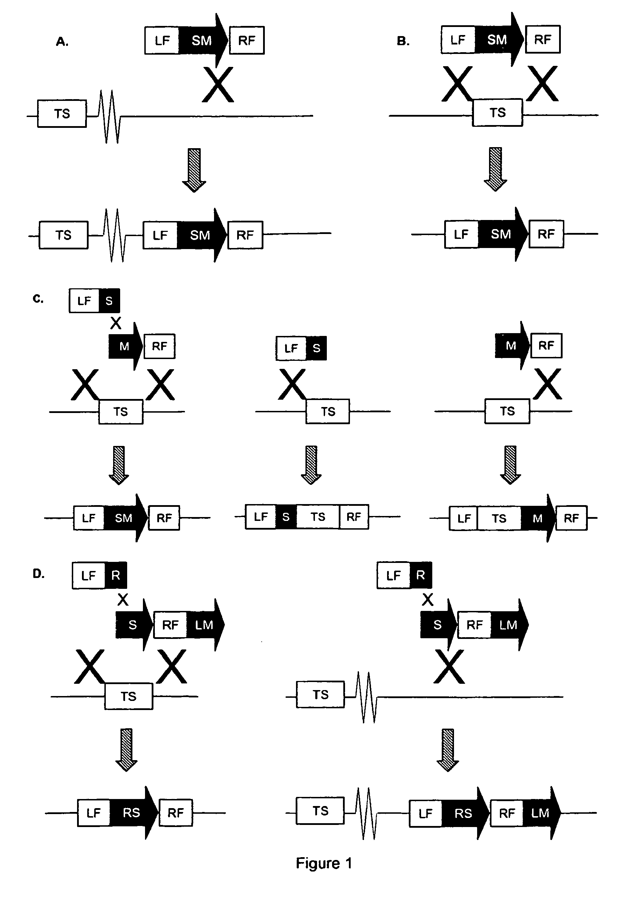

Eukaryotic cells have at least two separate pathways (one via non-homologous and one via homologous recombination; see Figures 1A and 1B, respectively) through which nucleic acids (in particular of course DNA) can be integrated into the host genome. The yeast *Saccharomyces cerevisiae* is an organism with a preference for homologous recombination (HR). The ratio of homologous to non-homologous recombination (HR/NHR) of this organism may vary from about 0.9 to 1. Contrary to *Saccharomyces cerevisiae,* most higher eukaryotic cells (including fungal, plant and mammalian) cells have a preference for non-homologous recombination (NHR). Among these, the HR/NHR ratio ranges between 0.0001 and 0.5. In such organisms, the targeted integration frequency is rather low. Also, the length of homologous regions flanking a polynucleotide sequence to be integrated into the genome of such organisms has to be relatively long, for example at least 2,000 base pairs for disrupting a single gene. The necessity of such flanking regions represents a heavy burden when cloning the DNA construct comprising said polynucleotide and when transforming the organism with it. Moreover, neighboring genes which lie within those flanking regions can easily be disturbed during the recombination processes following transformation, thereby causing unwanted and unexpected side-effects.

Recently, several publications describe the inhibition of the very efficient Non-Homologous End-Joining (NHEJ) pathway, one of the pathway responsible for random integration of polynucleotides in cells, as a method for improving the HR/NHR ratio (see for example Ninomiya et al, 2004, Proc. Natl. Acad. Sci. USA 101:12248-12253; Krappmann et al, 2006, Eukaryot. Cell. 5:212-215). It is potentially a very powerful method, resulting in significant improvements (up to 60-fold) of gene targeting efficiency.

However, there are still some drawbacks to this method. Firstly, it does not work for all species. For example, mammalian cells deficient in *ku70,* one of the components of the NHEJ pathway, have been isolated (Pierce et al, 2001, Genes and Development 15: 3237-3242). These mutants have a six-fold higher homology-directed repair frequency, but no increase in the efficiency of homology-directed targeted integration. Secondly, although it has a positive effect on the HR/NHR ratio in several fungal species (see for example Ninomiya et al., 2004; Krappmann et al., 2006), in most cases it is limited to 60-90% correct gene targeting. However, all the examples in literature are with species that have moderately high HR/NHR ratio's of 0.2-0.5 in the parent cells already. This is an acceptable improvement for working with one or several genes, but not for a High Throughput genome wide analysis and/or modification of gene function. In the individual cases were 100% correct transformants were obtained this involves long flanking regions, which also is not amenable for a High Throughput genome wide analysis and/or modification of gene function. Thirdly, to obtain such strains with improved HR/NHR ratios, one has to modify the recombination machinery of the host cell and this can lead to unwanted side effects (see for example Celli et al, 2006, Nat Cell Biol, 8: 885-890).

The HR/NHR ratio can also be improved by over expressing components of the HR pathway. An example of this method is given by Shaked et al. (2005, Proc Natl. Acad. Sci. USA. 102:12265-12269). They show that by over expression of yeast RAD54 the HR frequency can be improved a 100-fold. Still, this results in only 1-10% correct transformants, which makes this method not amenable for a High Throughput genome wide analysis and/or modification of gene function.

Another method is the so-called bipartite gene-targeting method (Nielsen et al., 2006, 43: 54-64; see Figure 1 C). This method is using two overlapping non-functional parts of a selection marker. Upon correct homologous recombination the selection marker becomes functional. They tested the method in the fungal species with the most efficient homologous recombination system, *Aspergillus nidulans,* with 24% correct gene targeting in WT cells. The method results in a 2.5-fold improvement over the standard method, but even in *Aspergillus nidulans* only 62% of the transformants obtained is correct. Also, rather long flanking regions are used to obtain correct targeting. This is an acceptable improvement for working with one or several genes, but not suitable for a High Throughput genome wide analysis and/or modification of gene function.

Liu et al. (2001, J. Bacteriol., 183: 1765-1772) describe another method, which uses a second selection marker to enrich for transformants with targeted gene disruption in *Acremonium chrysogenum.* The method results in a 10-fold improvement over the standard method, but still only 8% of the transformants obtained is correct.

Still another method is described by Kang and Khang (US 2005/0181509). This is a variation on the method of Liu et al. (2001). Here they apply a negative selection marker, i.e. the herpes simplex virus thymidine kinase *(HSVtk)* gene, as the second selection marker. If the selection procedure would work correctly, polynucleotides that integrate at random in the genome would kill the cells as the *HSVtk* gene would convert the 5-fluoro-2'-deoxyuridine in the agar plates to a toxic compound. Again, this method increases the frequencies of correct targeting in cells, but it is limited to 50% of the cells. More importantly there is a very high percentage of false positives obtained (9-100%), which makes this method unsuitable for a High Throughput genome wide analysis and/or modification of gene function.

Kang and Khang (US 2005/0181509) describe testing of the diphtheria toxin A *(dtA)* gene. This gene has been applied in plants and mammalian cells as a second marker to increase the frequency of correct gene targeting to 1-2% (see for examples Terada et al, 2004, Plant Cell Rep. 22:653-659; Yagi et al, 1993, Anal. Biochem. 214:77-86). However, they failed to get this marker functional in fungal species.

Surprisingly, it was found that by combining a method that increases the HR/NHR ratio with the bipartite gene-targeting method, a correct gene targeting at 100% can be obtained in cells which normally have a preference for NHR. Such an unexpected high targeting frequency is significantly higher than can be expected from the individual contributions of both methods.

The present invention discloses a method to construct eukaryotic cells having a target sequence in a chromosomal DNA sequence replaced by a replacement sequence of interest comprising:
- modifying a parent eukaryotic cell with a preference for NHR to provide a eukaryotic cell having an increased HR/NHR ratio as compared to the parent cell,
- providing two sets of DNA molecules of which the first set comprises DNA molecules each comprising a first non-functional fragment of the replacement sequence of interest flanked at its 5'-side by a DNA sequence substantially homologous to a sequence of the chromosomal DNA flanking the target sequence and the second set comprises DNA molecules each comprising a second non-functional fragment of the DNA replacement sequence of interest overlapping with the first non-functional fragment and flanked at its 3'-side by a DNA sequence substantially homologous to a sequence of the chromosomal DNA flanking the target sequence, wherein the first and second non-functional fragments become functional upon recombination;
- transforming the modified eukaryotic cells with both sets of DNA molecules; and
- growing the cells to obtain transformed progeny cells having a DNA molecule from the first set recombined with a DNA molecule from the second set into a DNA molecule comprising a functional replacement sequence and having the target sequence replaced by the functional replacement sequence.

The parent eukaryotic cell with a preference for NHR may be any eukaryotic cell having a HR/NHR ratio ≤ 0.5, preferably ≤0.2, more preferably ≤ 0.1, most preferably ≤ 0.05. The exact ratio may vary between different loci in one species. Suitable loci for determining the ratios above are the *niaD* locus (and its homologues in all species) and the *KU70* locus (and its homologues in all species).

The eukaryotic cell is a eukaryotic cell with a preference for NHR. Preferably, the eukaryotic cell is a fungal, plant or animal cell. More preferably, the fungus is of the genus *Aspergillus, Penicillium, Acremonium, Trichoderma, Chrysosporium, Mortierella, Kluyveromyces* or *Pichia;* most preferably of the species *Aspergillus niger, Aspergillus nidulans, Aspergillus oryzae, Aspergillus terreus, Penicillium chrysogenum, Penicillium citrinum, Acremonium chrysogenum, Trichoderma reesei, Mortierella alpina, Chrysosporium lucknowense, Kluyveromyces lactis, Pichia pastoris* or *Pichia ciferrii.* More preferably, the plant is of the genus *Arabidopsis, Nicotiana, Solanum, Lactuca, Brassica, Oryza, Asparagus, Pisum, Medicago, Zea, Hordeum, Secale, Triticum, Capsicum, Cucumis, Cucurbita, Citrullis, Citrus, Sorghum;* most preferably of the species *Arabidopsis thaliana, Nicotiana tabaccum, Solanum lycopersicum, Solanum tuberosum, Solanum melongena, Solanum esculentum, Lactuca sativa, Brassica napus, Brassica oleracea, Brassica rapa, Oryza glaberrima, Oryza sativa, Asparagus officinalis, Pisum sativum, Medicago sativa, Zea mays, Hordeum vulgare, Secale cereale, Triticum aestivum, Triticum durum, Capsicum sativus, Cucurbita pepo, Citrullus lanatus, Cucumis melo, Citrus aurantifolia, Citrus maxima, Citrus medica, Citrus reticulata.* More preferably, the animal cell is of the genus *Homo, Rattus, Mus, Sus, Bos, Danio, Canis, Felis, Equus, Salmo, Oncorhynchus, Gallus, Meleagris, Drosophila, Caenorhabditis;* most preferably of the species *Homo sapies, Rattus norvegicus, Mus musculus, Sus scrofa, Bos taurus, Danio rerio, Canis lupus, Felis catus, Equus caballus, Salmo salar, Oncorhynchus mykiss, Gallus gallus, Meleagris gallopavo, Drosophila melanogaster, Caenorhabditis elegans.*

The HR/NHR ratio is determined by analysis of a set of transformants to determine which part of the transformants has correct gene targeting (HR) and which part of the transformants has incorrect recombination (NHR). Several methods are available to obtain these data:
(i) phenotypic analysis; if the homologous target locus has an easily detectable phenotype, like the *niaD* locus (if deleted the cells become resistant to chlorate), all transformants may be tested on suitable discriminating media, for instance with and without chlorate, to obtain the frequency of HR and NHR events.
(ii) PCR analysis; DNA isolated from transformants may be subjected to PCR with one primer annealing outside the flanking sequences and one primer annealing within the replacement sequence. If amplification of the expected size is obtained, this confirms a HR event. If no fragment or a fragment of the wrong size is obtained, a control PCR may be done with both primers annealing to the target locus to be replaced. If amplification of the expected size is obtained, this confirms NHR events.
(iii) Southern blot analysis; DNA isolated from transformants is digested with restriction enzymes that generate different hybridization patterns dependent on a HR or NHR event, or on the wild type situation.

The preferred method for quickly scanning many transformants is PCR.

Eukaryotic host cells having an increased HR/NHR ratio as compared to a parent cell may be obtainable by modifying the parent eukaryotic cell by increasing the efficiency of the HR pathway and/or by decreasing the efficiency of the NHR pathway. The HR/NHR ratio thereby is increased at least 2 times, preferably at least 4 times, more preferably at least 10 times, even more preferably at least 25 times.

In the context of the invention, the HR pathway is defined as those genes and elements being involved in the control of the targeted integration of polynucleotides into the genome of a host, said polynucleotides having a certain homology with a certain predetermined site of the genome of a host to where the integration is targeted. The NHR pathway is defined as all genes and elements being involved in the control of the integration of polynucleotides into the genome of a host irrespective of the degree of homology of the polynucleotides with a genomic sequence of the host.

Increasing the efficiency of the HR pathway and/or decreasing the efficiency of the NHR pathway may be effectuated by up and/or down regulating the expression of genes involved in HR and/or NHR. The expression level of a DNA sequence, e.g. a gene, is up or down regulated, respectively, when the expression level of this DNA sequence in the obtained eukaryotic cell is higher or lower, respectively, than the expression level of the same DNA sequence in the parent eukaryotic cell, preferably at least 2 times higher or lower, more preferably at least 4 times higher or lower, even more preferably at least 10 times higher or lower. When down regulated, the expression of the DNA sequence most preferably is not detectable.

The up and/or down regulation of the expression level of a DNA sequence may be monitored by quantifying the amount of corresponding mRNA present in a cell by Northern blotting (see Molecular Cloning: A Laboratory Manual, Sambrook et al., New York: Cold Spring Harbour Press, 1989) and/or the amount of corresponding protein present by Western blotting. mRNA and protein levels may also be measured by 'omics' techniques like transcriptomics and/or proteomics. The difference in mRNA amount may also be quantified by DNA array analysis (Eisen, M. B. and Brown, P. O. DNA arrays for analysis of gene expression. Methods Enzymol. 1999: 303: 179-205).

The up and/or down regulation of the expression level of a DNA sequence may be obtained by subjecting the parent eukaryotic cell to recombinant genetic manipulation techniques and/or classical mutagenesis techniques. Up regulation of the expression of a DNA sequence using recombinant genetic manipulation techniques preferably comprises over expression of the DNA sequence. Down regulation of the expression of a DNA sequence using recombinant genetic manipulation techniques preferably comprises inactivation of the DNA sequence. Inactivation may be done by replacing the DNA sequence by a non-functional variant thereof or by deleting part or all of the DNA sequence from the genome.

In one embodiment, the up and/or down regulation of the expression level of a DNA sequence may be inducible. This can be achieved by replacing the endogenous regulatory regions of the DNA sequence, i.e. a gene encoding the component involved in HR and/or NHR, by new regulatory regions, preferably comprising a repressible or inducible promoter, more preferably a promoter that can be switched on/off, for instance by glucose repression, ammonia repression, and/or pH repression. Examples of fungal glucose-repressed promoters are the *Penicillium chrysogenum* pcbAB promoter (Martin et al, 1999, Antonie Van Leeuwenhoek. 75:21-31) or the *Aspergillus niger* glucoamylase promoter. Examples of on/off switchable promoters are described in Belli et al (1998, Nucl. Acid Res. 26: 942-947: an activator/repressor dual system allows tight tetracycline-regulated gene expression in budding yeast) and Shimizu et al (2002, Nat. Biotech. 20:1041-1044: a light-switchable gene promoter system).

A suitable eukaryotic cell may subsequently be selected by monitoring the expression level of the relevant DNA sequence. Optionally, the eukaryotic cell may be selected by measuring the efficiency of the NHR and/or of the HR pathways and/or the HR/NHR ratio. In the context of the invention, the efficiency of the HR pathway of a filamentous fungus may be measured by the efficiency of the targeted integration of a given polynucleotide sequence into a predetermined site in the genome of the filamentous fungus using given homology region(s). In the context of the invention, the efficiency of the NHR pathway of a filamentous fungus may be measured by the efficiency of the non targeted integration of a given polynucleotide sequence in the genome of the filamentous fungus irrespective of any homology region(s).

In a preferred embodiment, eukaryotic cells having an increased HR/NHR ratio as compared to a parent cell are obtainable by decreasing the efficiency of the NHR pathway. This can be achieved by down regulating the expression of a gene involved in NHR in the eukaryotic cell as comparison to the expression of said gene in the parent eukaryotic cell measured under identical conditions.

According to a preferred embodiment, the eukaryotic cell having an increased HR/NHR ratio as compared to a parent cell is deficient in at least one of its endogenous genes that are equivalents of the following yeast genes involved in the NHR pathway: KU70, KU80, RAD50, MRE11, XRS2, LIG4, LIF1,NEJ1 and SIR4 (van den Bosch et at, 2002, Biol. Chem. 383: 873-892 and Allen et al, 2003, Mol, Cancer Res. 1:913-920). An example of equivalents of the yeast KU70 and KU80 genes, respectively, are the genes *hdfA* or *mus51* and *hdfB* or *mus52,* respectively, from filamentous fungi (see WO05095624 and Ninomiya et al, 2004). Mammalian equivalents of the yeast genes are also known as KU70 or KU80 genes (see for example Pierce et al, 2001).

The eukaryotic cell having an increased HR/NHR ratio as compared to a parent cell as specified herein above is preferably used as a host cell for transformation using the DNA molecules as specified herein below.

Each member of the two sets of DNA molecules comprises a non-functional replacement sequence, the non-functional replacement sequence of the members of the first set overlapping with the non-functional remplacement sequence of the of the members other set. With the term "overlapping" as used in this invention is meant that two non-functional DNA sequences together comprising the replacement sequence have a common DNA sequence, localized at the 3'-end of the first sequence and the 5'-end of the second sequence. The overlapping sequence typically may be about 30-50% of the functional replacement sequence. Conveniently, the overlapping sequence may have a length of 0.03 to 2 kb.

The two sets of DNA molecules are transformed into a suitable host cell with an improved HR/NHR ratio as described above, using techniques commonly known in the art. The DNA molecules are preferably linear molecules. Optionally, co-transformation may be done with a further set of DNA molecules comprising a selection marker.

Inside the cell, recombination between the two non-functional replacement sequences can occur at the overlapping part of the replacement sequence. Such recombination will result in one DNA fragment for insertion into the chromosomal DNA. This fragment comprises a 5'-flanking sequence, the functional replacement sequence and a 3'-flanking sequence. The substantially homologous flanking sequences further can recombine with their corresponding target sequences on the chromosome. Both recombination events are catalyzed by the enzymes of the HR pathway which are the predominantly present recombination enzymes in cells with an improved HR/NHR ratio. The homologous integration event occurs at the target sequence in the host chromosome by a double cross-over event at the substantially homologous sequences flanking the replacement and targeting sequence. The integration event ensures that the concurrently recombined replacement sequence is stably integrated in the chromosome.

The term "substantially homologous" refers to a DNA sequence flanking the replacement sequence having a degree of identity to a chromosomal DNA sequence flanking the target sequence of at least 80%, preferably at least 90%, more preferably 100%. The degree of required identity may thereby depend on the length of the substantially homologous sequence. A flanking having a length lower than about 0.1 kb may need a higher degree of identity than 80% with the chromosomal DNA flanking the target sequence. The use of the cells with an improved HR/NHR ratio advantageously allows a reduction of the length of the flankings, for instance to about 0.03-0.1 kb. Larger flankings may also be used, for instance to about 2-3 kb.

The nature of the replacement sequence may vary depending on the intended use. The replacement sequence may for instance confer a screenable and/or selectable phenotype to the eukaryotic cell, in which case the replacement sequence comprises a selection marker. Preferably, the selection marker is a positive selection marker. A preferred positive selection marker is the *amdS* gene. Other examples of such selection markers are Green Fluorescent Protein (eGFP), Yellow Fluorescent Protein (eYFP), Cyan Fluorescent Protein (eCFP), Blue Fluorescent Protein (eBFP), Red Fluorescent Protein (RFP), β-galactosidase (β-gal), glucoronidase (GUS), tn5-ble (phleomycin^{R}), hyg (hygromycin^{R}), kan (kanamycin^{R}), gen (G418^{R}). A selection marker as replacement sequence preferably is used when the target sequence needs to be inactivated.

As part of the present invention, it is shown that by supplying the selectable marker as two overlapping, but non-functional fragments, cells with an improved HR/NHR ratio show a surprisingly high efficiency in recombining these fragments into a functional and thus selectable marker again.

The replacement sequence may also be a modified version of the target sequence, for instance to provide for altered regulation of a sequence of interest or expression of a modified gene product with altered properties as compared to the original gene product. The replacement sequence may also constitute additional copies of a sequence of interest being present in the genome of a eukaryotic cell, to obtain amplification of that sequence of interest. The replacement sequence may be a sequence homologous or heterologous to the eukaryotic cell of interest. It may be obtainable from any suitable source or may be prepared by custom synthesis.

When the replacement sequence does not confer a screenable and/or selectable phenotype to the cell, differential fluorescent labeling may be applied in combination with the use of a fluorescence activated cell sorter to select cells wherein the recombined replacement sequence is correctly integrated. The two sets of DNA molecules comprising the overlapping DNA fragments as described above are labeled with two different fluorescent dyes for each set, transformed and selected as described in WO 2005/040186. To be able to select for cells that have the two overlapping DNA molecules recombined, the fluorescent dye of the first fragment is chosen in such a way that its excitation spectrum does neither overlap with the excitation nor with the emission spectrum of the fluorescent dye of the second fragment and its emission spectrum significantly overlaps with the excitation spectrum of the second fragment. After recombination and integration in the genome by homologous recombination, the two different fluorescent labels are in close proximity of each other (in fact physically linked) so one can use Fluorescence Resonance Energy Transfer (FRET) in which the emission energy of the first dye is used to excite the second dye. Cells are sorted when they show emission of the second dye. Examples of such dye pairs are Alexa Fluor 488 and Alexa Fluor 594, DAPI and Alexa Fluor 546, DAPI and propidium iodide.

Another way to implement targeted integration of a replacement sequence of interest which does not confer a screenable and/or selectable phenotype to the cell is to include the replacement sequence of interest in the first and/or second DNA molecule as an additional replacement sequence adjacent to the first and/or second non-functional selection marker fragment. The additional replacement sequence may be inserted at the 3'-side of the 3'-non-functional selection marker fragment and/or at the 5'-side of the 5'-non-functional selection marker fragment. The functional selection marker that is formed upon recombination and integration in the chromosome may optionally be deleted following the strategy of the construction of selection marker-free cells as disclosed in EP 635574.

In one particular embodiment of the present invention, the method of the invention is combined with the use of a further selectable marker physically linked to one of the two non-functional fragments used in the method of the invention. In this embodiment, the further selectable marker is localized 5'- of the 5'-flanking or 3'- of the 3'-flanking (see Figure 1 D). After recombination at the overlapping part of the replacement sequence inside the cell, one DNA fragment is obtained for insertion in the chromosomal DNA, comprising for instance a 5'-flanking sequence; the replacement sequence; a 3'-flanking sequence and the DNA fragment encoding the further selectable marker. However, only a non-homologous recombination event will lead to the actual integration of the further selectable marker fragment. A homologous integration event by a double cross-over at the flanking sequences will exclude the selectable marker fragment. The further selectable marker may be a selection marker as described above.

In a preferred embodiment the said further selectable marker is a lethal selection marker, which confers a lethal phenotype to the transformed cell. Such a lethal phenotype may be achieved in various ways. A lethal phenotype for instance may directly be achieved when the lethal selection marker encodes a compound that is toxic to the cell, e.g. diphtheria toxin A or suppressor tRNA's. A lethal phenotype may also conditionally be achieved when the lethal selection marker encodes an enzyme that converts a particular substrate into a toxic compound. Examples of such conditional lethal markers, in combination with a substrate that can be converted into a toxic compound, are: nitrate reductase *(niaD* gene) in combination with chlorate, orotidine-5'-monophosphate decarboxylase *(pyrG* gene) in combination with fluoro-orotic acid, acetamidase *(amdS* gene) in combination with fluoro-acetamide, or thymidine kinase (tk gene) in combination with 5-fluoro-2'-deoxyuridine. The use of such a further selectable marker is particularly useful when the replacement sequence itself has no screenable and/or selectable trait and fluorescence is used to select for the recombination between the two overlapping fragments of the replacement sequence.

According to the invention, the target sequence may be any sequence of interest. For instance, the target sequence may be a sequence of which the function is to be investigated by inactivating or modifying the sequence. The target sequence may also be a sequence of which inactivation, modification and/or over expression is desirable to confer a eukaryotic cell with a desired phenotype. Suitable target sequences may be regulatory sequences and/or coding sequences.

The replacement sequence and/or the selectable marker may be provided as part of an expression cassette. An expression cassette comprises regulatory sequences operably linked to a coding sequence. The term "operably linked" refers to a juxtaposition wherein the components described are in a relationship permitting them to function in their intended manner. A regulatory sequence such as a promoter, an enhancer or another expression regulatory signal "operably linked" to a coding sequence is positioned in such a way that expression of a polypeptide from its coding sequence is achieved under conditions compatible with the regulatory sequences.

The regulatory sequences of the expression cassette preferably are heterologous to the chromosome of the eukaryotic cell of interest, i.e. the regulatory sequences are from a different eukaryotic species than the eukaryotic cell of interest to be transformed. The use of a homologous regulatory sequence may result in a targeted integration event at a chromosomal site corresponding to the homologous regulatory sequence. Such an integration event is undesirable because it decreases the percentage correct targeting to the site comprising the targeting sequence.

By using eukaryotic cells with an increased HR/NHR ratio, the method of the invention advantageously ensures an increased targeting of a polynucleotide to a chromosomal target site of interest. The method of the invention further advantageously allows the provision of transformed eukaryotic cells that are enriched in cells wherein the correct targeted integration event has occurred. In particular, at least 50% of the transformed clones have the replacement sequence targeted to the target sequence in the chromosome, as compared to 0.1-2% in a transformation with a targeting construct without the use of the bipartite gene-replacement method and without a strain with an improved HR/NHR ratio.

### Description of the Figures

Figure 1 shows the different recombination pathways. A. Non-Homologous Recombination, resulting in a random integration event. In this case the replacement sequence is a selectable marker (SM). B. (Double) Homologous Recombination, resulting in a targeted integration event. In this case the target sequence (TS) is replaced by a SM. C. The bipartite gene-replacement method, resulting in a combined recombination and targeted integration event. In this case two non-functional overlapping SM fragments are recombined and targeted to the TS. If recombination does not occur, integration (either homologous or non-homologous) does not lead to selectable phenotype. D. The bipartite gene-replacement method combined with a second DNA fragment encoding a selection marker, resulting in a combined recombination and targeted integration event. In this case two non-functional overlapping replacement sequence (RS) fragments are recombined and targeted to the TS. If non-homologous recombination does occur the second DNA fragment integrates and can be used to deselect such cells.

Legend: LF = 5'-homologous flanking region of the target sequence; SM = selection marker gene cassette; RF = 3'-homologous flanking region of the target sequence; TS = target sequence; RS = replacement sequence; LM = second DNA fragment encoding a selection marker. The 'X' represents recombination events.

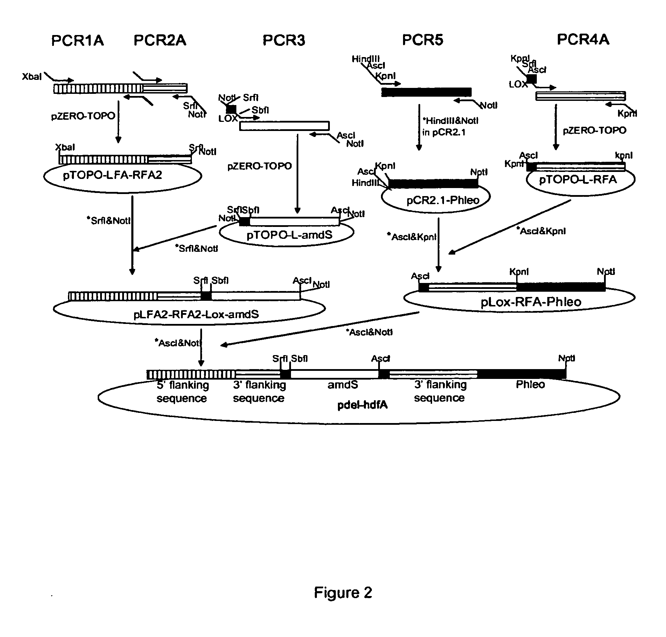

Figure 2 shows the cloning scheme of plasmid pdel-hdfA. The details of the five PCR reactions PCR1A, PCR2A, PCR3, PCR4A and PCR5 are specified in example 1. Each intermediate plasmid name and relevant restriction enzyme sites are indicated. Legend: = illustrates restriction digest with enzymes; boxes with vertical lines = 5'-homologous flanking sequence; boxes with horizontal lines = 3'-homologous flanking sequence; white boxes = amdS selection marker cassette; large black boxes = phleomycin selection marker cassettes; small black boxes = recombination sequences.

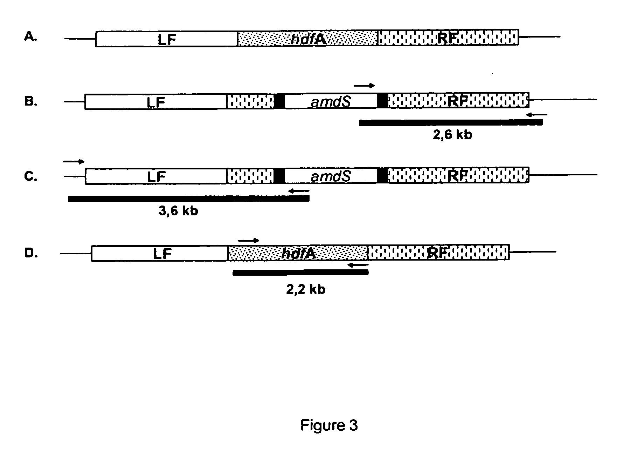

Figure 3 is the schematic representation of the PCR controls to determine if the P. chrysogenum *hdfA* gene was deleted correctly. A. The WT locus. B. Correct gene targeting would produce a 2.6 kb fragment with oligonucleotide SEQ ID NO 11 and SEQ ID NO 12; the WT should not produce a fragment. C. Correct gene targeting would produce a 3.6 kb fragment with oligonucleotide SEQ ID NO 13 and SEQ ID NO 14; the WT should not produce a fragment. D. Correct gene targeting would not produce a fragment with oligonucleotide SEQ ID NO 15 and SEQ ID NO 16; the WT should produce a 2.2 kb fragment. Legend: LF = 5'-homologous flanking sequence; amdS = amdS selection marker cassette; RF = 3'homologous flanking sequence; hdfA = Penicillium chrysogenum hdfA gene (yeast KU70 homologue). The arrows indicate the annealing position of the oligonucleotides as specified in the text of example 1. The thick lines indicate the PCR fragment amplified in the PCR reactions as specified in the text of example 1.

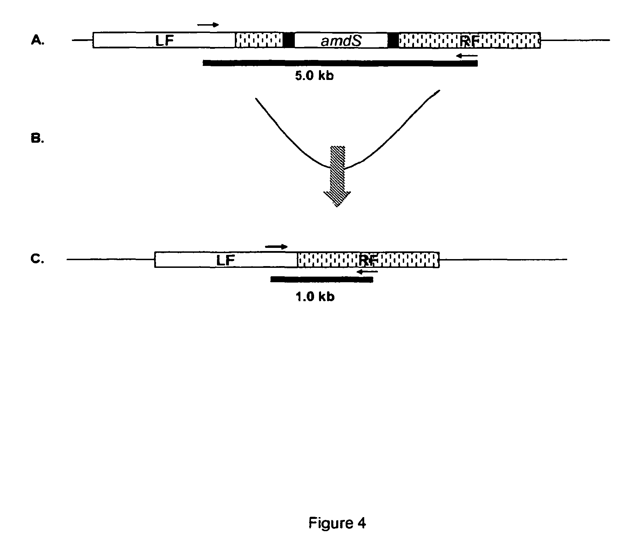

Figure 4 is the schematic representation of the PCR controls to determine if the marker-free version of the P. chrysogenum *hdfA* deletion mutant was obtained. A. *hdfA* deletion with the amdS marker still present should produce a 5 kb fragment with oligonucleotide SEQ ID NO 17 and SEQ ID NO 18. B. Schematic representation of the recombination event selected, enabling the cells to become resistant versus fluoroacetamide. C. Marker-free *hdfA* deletion should produce a 1 kb fragment with oligonucleotide SEQ ID NO 17 and SEQ ID NO 18. Legend: LF = 5'-homologous flanking sequence; amdS = amdS selection marker cassette; RF = 3'-homologous flanking sequence; The arrows indicate the annealing position of the oligonucleotides as specified in the text of example 1. The thick lines indicate the PCR fragment amplified in the PCR reactions as specified in the text of example 1.

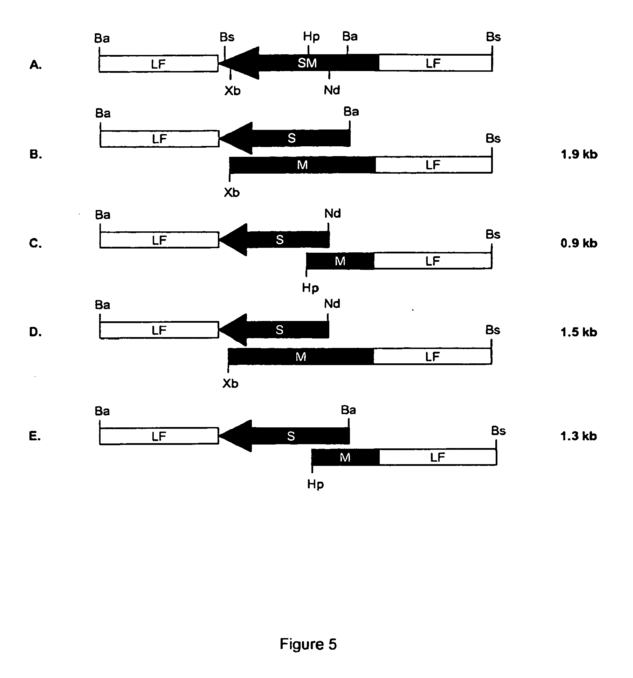

Figure 5 is the schematic representation of the different overlapping fragments used in the bipartite gene-replacement. A. the intact DNA fragment; B. Two fragments with 1.9 kb overlap; C. Two fragments with 1.0 kb overlap; D. Two fragments with 1.5 kb overlap; E. Two fragments with 1.3 kb overlap.

Legend: Ba = BamHI; Bs = BstXl; Nd= Ndel; Hp = Hpal; LF = 5'-homologous flanking region of the target sequence; SM = selection marker gene cassette; RF = 3'-homologous flanking region of the target sequence.

### EXAMPLES

### General materials and methods

Standard procedures were carried out as described in Sambrook et al. (1989, Molecular cloning: a laboratory manual, 2nd Ed., Cold Spring Harbor Laboratory Press, Cold Spring Harbor, New York). DNA for plasmid construction was amplified using a proofreading polymerase, Phusion (Finnzymes) following manufacturer's protocol; while verification of constructed strains and plasmids was achieved by using Taq polymerase. Restriction enzymes were from Invitrogen or New England Biolabs. For routine cloning, *Escherichia coli* strains Top10 and DH10B (Invitrogen) were employed. The Gateway system of Invitrogen was applied according to manufacturer's manuals. Verification of the constructed plasmids was carried out by restriction analysis and sequencing.

### Example 1

### Deletion of the hdfA gene of Penicillium chrysogenum

### Vector construction

To delete the chromosomal copy of the *Penicillium chrysogenum hdfA* gene (see WO05095624), the fungal equivalent of the yeast KU70 gene involved in the Non-Homologous End-Joining pathway, the integration vector pdeI-*hdfA* was constructed. To this end 2500 bp genomic DNA fragments both directly upstream and directly downstream the *hdfA* gene were PCR amplified. The upstream region (alias left flanking; PCR1A in Figure 2) was PCR amplified from genomic DNA using the oligonucleotides of SEQ ID NO 1 and SEQ ID NO 2, thereby introducing an Xbal site at the far left end. To facilitate later removal of the *amdS* selection marker a 1000 bp repeat surrounding this cassette was introduced in the construct, by PCR amplification of the first 1000 bp downstream of the *hdfA* gene from genomic DNA and fusing this to the left flanking (PCR2A in Figure 2). For this the oligonucleotides of SEQ ID NO 3 and SEQ ID NO 4 where used, introducing SrfI and NotI sites at the right end. The whole fragment (3.5 kb) was cloned in pZERO-TOPO (Invitrogen), yielding plasmid pTOPO-LFA-RFA2. The amdS selection marker cassette was PCR amplified from pHELY-A1 (see WO 04106347) using oligonucleotides of SEQ ID NO 5 and SEQ ID NO 6, introducing Notl, SrfI and Sbfl sites on the left end and AscI and NotI sites on the right end (PCR3 in Figure 2). Also this fragment was cloned in to pZERO-TOPO (i.e. yielding plasmid pTOPO-L-amdS) and subsequently the SrfI-NotI fragment was recloned into pTOPO-LFA-RFA2, yielding plasmid pLFA2-RFA2-Lox-amdS. The downstream region (alias right flanking; PCR4A in Figure 2) was PCR amplified from genomic DNA using the oligonucleotides of SEQ ID NO 7 and SEQ ID NO 8, thereby introducing KpnI, SrfI and AscI sites at the left end and a Kpnl site on the right end. Again the fragment was cloned in to pZERO-TOPO, yielding plasmid pTOPO-L-RFA. To facilitate later an efficient screening for targeted integration at the *hdfA* locus, the phleomycin resistance cassette was PCR amplified (PCR5 in Figure 2; Phleo) from pAMPF21 (Fierro et al, 1996, Curr Genet 29: 482-489) using the oligonucleotides of SEQ ID NO 9 and SEQ ID NO 10, thereby introducing Hindlll, AscI and KpnI sites at the left end and NotI at the right end. The fragment was digested with HindIII and NotI and cloned in to pCR2.1, yielding pCR2.1-Phleo. The right flanking was isolated from pTOPO-L-RFA after digestion with AscI and Kpnl; and cloned into pCR2.1-Phleo, yielding plasmid pLox-RFA-Phleo. The complete deletion construct of *hdfA* was obtained after isolating the AcsI-NotI fragment of pLox-RFA-Phleo and ligating this in pLFA-RFA2-Lox-amdS digested with AcsI-NotI, yielding plasmid pdeI-*hdfA* (Figure 2).

### Deletion of chromosomal copy

The deletion fragment was isolated from plasmid pdeI-*hdfA* as a SfiI fragment and transfected to *Penicillium chrysogenum* protoplasts. *Penicillium chrysogenum* protoplasts were produced according to standard protocols (see for examples Cantoral et al., 1987, Biotechnology 5: 494-497; Swinkels et al., 1997, WO 97/06261) however Glucanex™ (Sigma) was applied as lysing enzyme. After transformation the protoplasts were plated out on selective regeneration agar plates with acetamide as the sole nitrogen source (for an exact description of the media, see Swinkels et al., 1997). Acetamide utilizing colonies were transferred to fresh acetamide plates (without saccharose to induce sporulation) and 300 of these were subsequently screened for phleomycin sensitivity (on mineral medium agar as described for *Penicillium chrysogenum* in US 2002/0039758, without phenyl acetic acid but supplemented with 15 g/L agar to solidify and 50 mg/L phleomycin). The thus obtained transformants should be enriched for targeted integration at the *hdfA* locus as the random transformants would be phleomycin resistant. Out of the 300 acetamide utilizing transformants only 9 were phleomycin sensitive. This suggested that 3% of the original transformants were putative correct transformants, i.e. targeting towards the *hdfA* could be successful. This is in line with values reported for filamentous fungi were in WT situations most of the transformants are the result of random integration. By adding the screening for phleomycin sensitivity we could efficiently deselect these and put more effort on a limited number (i.e. 9) of putative *hdfA* mutants.

### Confirmation of hdfA deletion

Six of the 9 putative *hdfA* mutants were screened with colony PCR to determine if the deletion was correct. To this end a piece of the sporulated colony was resuspended in 50 µl of water and boiled for 10 minutes at 98 degrees Celsius. The cell debris was spun down and one µl of the supernatant was used in 3 PCR reactions (Figure 3). Using a primer set with the forward primer annealing at the terminator of the amdS cassette and the reverse primer annealing downstream of the right flank used in the deletion cassette only transformants with the correct integration event should give a 2.6 kb fragment (Figure 3B). The PCR reaction with oligonucleotides SEQ ID NO 11 and SEQ ID NO 12 showed that all 6 candidates had the correct integration at this site. Using a primer set with the forward primer annealing upstream of the left flank used in the deletion cassette and the reverse primer annealing at the gpdA promoter of the amdS cassette only transformants with the correct integration event should give a 3.6 kb fragment (Figure 3C). The PCR reaction with oligonucleotides SEQ ID NO 13 and SEQ ID NO 14 showed that only one of the candidates had the correct integration at this site. Using a primer set with the forward primer starting at the ATG of the *hdfA* gene and the reverse primer starting just in front of the STOP codon of the *hdfA* transformants with the correct integration event should not produce a fragment. The PCR reaction with oligonucleotides SEQ ID NO 15 and SEQ ID NO 16 showed that single candidate left after the first two PCRS indeed gave no band, while in the other five candidates *hdfA* could still be amplified, indicating that in this latter group an odd recombination took place at the *hdfA* locus. However, strain SA1 was confirmed to be a real *hdfA* deletion strain. So in conclusion 1 out of the 6 pre-selected transformants has a correct *hdfA* gene replacement, meaning that the HR/NHR ratio in this experiment is 0.005 (1 out of 200 primary transformants).

### Obtain marker-free hdfA mutant

In order to obtain a clean *hdfA* mutant the amdS selection marker cassette had to be removed. To facilitate this, a 1000 bp repeat was introduced during cloning (Figure 2). This 1000 bp is exactly the same as the first 1000 bp of the right flanking region used in the deletion construct. Thus, if a recombination would take place at this 1000 bp a clean *hdfA* deletion should be obtained in which the flanking regions are retained and the complete *hdfA* locus will be removed. The amdS selection marker has the advantage that one can select for the presence (i.e. enabling growth on acetamide as the sole nitrogen source) as well on the absence (i.e. fluoroacetamide would generate toxic fluorine in the presence of amdS) of the gene. Spores of strains SA1 were plated on fluoroacetamide containing agar plates (see WO 9706261) and growing colonies were isolated and checked with colony PCR. To this end PCR template material was obtained as described above and the PCR was run using the oligonucleotides SEQ ID NO 17 and SEQ, ID NO 18. In case of SA1 this should give a fragment of 5 kb, while in the recombined situation (i.e. marker-free *hdfA* deletion) this should give a fragment of 1 kb (Figure 4). Ten fluoroacetamide resistant colonies were checked and all showed the 1 kb band. On top of this none of these 10 candidates was able to grow on acetamide plates, confirming that the recombination took place: the amdS gene was removed and these are true *hdfA* marker-free deletion strains (AS1 to AS10).

### Phenotype of the marker-free hdfA mutant

To determine if the *hdfA* mutants had no obvious phenotype, 5 items were checked: penicillin V production, morphology, growth rate, sporulation and phleomycin sensitivity. Penicillin V production was checked in liquid medium as described in US 2002/0039758. Four strains were checked: the WT, SA1 *(hdfA* mutant containing amdS), AS1 and AS2 *(hdfA* mutants both marker-free). The productivities were respectively 100%, 114%, 105% and 100%. So, the *hdfA* deletion causes no apparent change towards penicillin production. Morphology was checked by growing the mutants on plate. No significant difference could be observed versus the WT. So, the *hdfA* deletion causes no apparent change towards morphology. Growth rate was checked during the Penicillin V productivity tests as described above by measuring the OD600. No significant difference could be observed versus the WT. So, the *hdfA* deletion causes no apparent change towards growth rate. Sporulation was checked by visually comparing the coloring and structure of the colonies on agar plates. No significant difference could be observed versus the WT. So, the *hdfA* deletion causes no apparent change towards sporulation. As the *hdfA* gene product possibly has a role in repair mechanisms the sensitivity versus phleomycin, a known inducer of DNA breaks, was check. This was done by testing the growth on mineral medium agar plates as described for *Penicillium chrysogenum* in US 2002/0039758, without phenyl acetic acid but supplemented with 15 g/L agar to solidify and 0-50 mg/L phleomycin. No significant difference could be observed versus the WT. So, the *hdfA* deletion causes no apparent change towards phleomycin sensitivity. Finally, we concluded that the *hdfA* mutant has no apparent phenotype and can be used for further studies.

### Example 2

### Deletion of the hdfB gene of Penicillium chrysogenum

### Vector construction

To delete the chromosomal copy of the *Penicillium chrysogenum hdfB* gene (see WO 05095624), the fungal equivalent of the yeast KU80 gene involved in the Non-Homologous End-Joining pathway, the integration vector pdeI*-hdfB* was constructed. The construction was generally the same as outlined in Figure 1 for the *hdfA* gene. First, two 2500 bp genomic DNA fragments directly upstream and directly downstream the *hdfB* gene were PCR amplified. The upstream region (alias left flanking) was PCR amplified from genomic DNA using the oligonucleotides of SEQ ID NO 19 and SEQ ID NO 20, thereby introducing an XbaI site at the far left end. To facilitate later removal of the *amdS* selection marker a 1000 bp repeat surrounding this cassette was introduced in the construct, by PCR amplification of the first 1000 bp downstream of the *hdfB* gene from genomic DNA and fusing this to the left flanking. For this the oligonucleotides of SEQ ID NO 21 and SEQ ID NO 22 where used, introducing SrfI and NotI sites at the right end. The whole fragment (3.5 kb) was cloned in pZERO-TOPO (Invitrogen), yielding plasmid pTOPO-LFB-RFB2. The SrfI-NotI fragment of pTOPO-L-amdS was recloned into pTOPO-LFB-RFB2, yielding plasmid pLFB2-RFB2-Lox-amdS. The downstream region (alias right flanking) of *hdfb* was PCR amplified from genomic DNA using the oligonucleotides of SEQ ID NO 23 and SEQ ID NO 24, thereby introducing KpnI, SrfI and AscI sites at the left end and a KpnI site on the right end. Again the fragment was cloned in to pZERO-TOPO, yielding plasmid pTOPO-L-RFB. This right flanking was isolated from pTOPO-L-RFB after digestion with AscI and Kpnl; and cloned into pCR2.1-Phleo, yielding plasmid pLox-RFB-Phleo. The complete deletion construct of *hdfB* was obtained after isolating the AcsI-NotI fragment of pLox-RFB-Phleo and ligating this in pLFB-RFB2-Lox-amdS digested with AcsI-NotI, yielding plasmid pdeI-*hdfB* (Figure 1).

### Deletion of chromosomal copy

The deletion fragment was isolated from plasmid pdel-*hdfb* as a Sfil fragment and transfected to *Penicillium chrysogenum* protoplasts that were produced according to standard protocols (see for example Cantoral et al., 1987, Biotechnology 5:494-497; Swinkels et al., 1997, WO97/06261) however Glucanex™ (Sigma) was applied as lysing enzyme. After transformation the protoplasts were plated out on selective regeneration agar plates with acetamide as the sole nitrogen source (for an exact description of the media, see Swinkels et al., 1997). Acetamide utilizing colonies were transferred to fresh acetamide plates (without saccharose to induce sporulation) and 375 of these were subsequently screened for phleomycin sensitivity (on mineral medium agar as described for *Penicillium chrysogenum* in US 2002/0039758, without phenyl acetic acid but supplemented with 15 g/L agar to solidify and 50 mg/L phleomycin). The thus obtained transformants should be enriched for targeted integration at the *hdfA* locus as the random transformants would be phleomycin resistant (outlined for *hdfA* in Figure 2). Out of the 375 acetamide utilizing transformants only 12 were phleomycin sensitive. This suggested that 3.2% of the original transformants were putative correct transformants, i.e. targeting towards the *hdfB* could be successful. This is in line with values reported for filamentous fungi were in WT situations most of the transformants are the result of random integration. By adding the screening for phleomycin sensitivity we could efficiently deselect these and put more effort on a limited number (i.e. 12) of putative *hdfB* mutants.

### Confirmation of hdfB deletion

Eight of the 12 putative *hdfA* mutants were screened with colony PCR to determine if the deletion was correct. To this end a piece of the sporulated colony was resuspended in 50 µl of water and boiled for 10 minutes at 98 degrees Celsius. The cell debris was spun down and one µl of the supernatant was used in 3 PCR reactions (similar set-up as outlined for *hdfA* in Figure 3). Using a primer set with the forward primer annealing at the terminator of the amdS cassette and the reverse primer annealing downstream of the right flank used in the deletion cassette only transformants with the correct integration event should give a 2.6 kb fragment. The PCR reaction with oligonucleotides SEQ ID NO 11 and SEQ ID NO 25 showed that all 8 candidates had the correct integration at this site. Using a primer set with the forward primer annealing upstream of the left flank used in the deletion cassette and the reverse primer annealing at the gpdA promoter of the amdS cassette only transformants with the correct integration event should give a 3.6 kb fragment. The PCR reaction with oligonucleotides SEQ ID NO 26 and SEQ ID NO 14 showed that 7 out of the 8 candidates had the correct integration at this site. Using a primer set with the forward primer starting at the ATG of the *hdfB* gene and the reverse primer starting just in front of the STOP codon of the *hdfB* transformants with the correct integration event should not produce a fragment. The PCR reaction with oligonucleotides SEQ ID NO 27 and SEQ ID NO 28 showed that from the 7 candidates left after the first two PCRs 5 indeed gave no band, while in the other three candidates (parts of) *hdfB* could still be amplified, indicating that in this latter group an odd recombination took place at the *hdfB* locus. However, strains SB2, SB3, SB4, SB5 and SB12 were confirmed to be real *hdfB* deletion strains. So in conclusion 5 out of the 8 pre-selected transformants has a correct *hdfB* gene replacement, meaning that the HR/NHR ratio in this experiment is 0.02 (5 out of 250 primary transformants).

### Obtain marker-free hdfB mutants

In order to obtain a clean *hdfB* mutant the amdS selection marker cassette had to be removed. To facilitate this, a 1000 bp repeat was introduced during cloning (see above). This 1000 bp is exactly the same as the first 1000 bp of the right flanking region used in the deletion construct. Thus, if a recombination would take place at this 1000 bp a clean *hdfB* deletion should be obtained in which the flanking regions are retained and the complete *hdfB* locus will be removed. The amdS selection marker has the advantage that one can select for the presence (i.e. enabling growth on acetamide as the sole nitrogen source) as well on the absence (i.e. fluoroacetamide would generate toxic fluorine in the presence of amdS) of the gene. Spores of the 5 *hdfB* strains were plated on fluoroacetamide containing agar plates (see WO 9706261) and growing colonies were isolated and checked with colony PCR. To this end PCR template material was obtained as described above and the PCR was run using the oligonucleotides SEQ ID NO 29 and SEQ ID NO 30. If amdS would be present this should give a fragment of 5.5 kb, while in the recombined situation (i.e. marker-free *hdfB* deletion) this should give a fragment of 1 kb (as outlined for *hdfA* in Figure 4). Ten fluoroacetamide resistant colonies were checked for each of the 5 *hdfB* strains and all derivatives showed the 1 kb band. On top of this none of these 50 derivatives was able to grow on acetamide plates, confirming that the recombination took place: the amdS gene was removed and these are true *hdfB* marker-free deletion strains.

### Phenotype of the marker-free hdfB mutant

As for the *hdfA* mutant we determined if the *hdfB* mutants had no obvious phenotypes. The same5 items were checked: penicillin V production, morphology, growth rate, sporulation and phleomycin sensitivity. Penicillin V production was checked in liquid medium as described in US 2002/0039758. Two marker-free derivatives were checked per amdS-positive *hdf8* strain and compared to the productivity of the WT strain (see Table 1).

**Table 1. Penicillin V productivities of hdf8 mutants (productivity of the WT set at 100)**

| AMDS | + | | - | | | |
|---|---|---|---|---|---|---|
| | Strain | Penicillin V | Strain | Penicillin V | Strain | Penicillin V |
| | SB2 | 108 | BS1 | 98 | BS2 | 100 |
| | SB3 | 106 | BS3 | 100 | BS4 | 102 |
| | SB4 | 100 | BS5 | 98 | BS6 | 101 |
| | SB5 | 116 | BS7 | 101 | BS8 | 97 |
| | SB12 | 92 | BS9 | 86 | BS10 | 88 |

The productivities all are around 100 +/-10% of the WT strains. So, the *hdfB* deletion causes no apparent change towards penicillin production. Morphology was checked by growing the mutants on plate. No significant difference could be observed versus the WT. So, the *hdfB* deletion causes no apparent change towards morphology. Growth rate was checked during the penicillin V productivity tests as described above by measuring the OD600. No significant difference could be observed versus the WT. So, the *hdfB* deletion causes no apparent change towards growth rate. Sporulation was checked by visually comparing the coloring and structure of the colonies on agar plates. No significant difference could be observed versus the WT. So, the *hdfB* deletion causes no apparent change towards sporulation.
As the *hdfB* gene product possibly has a role in repair mechanisms the sensitivity versus phleomycin, a known inducer of DNA breaks, was check. This was done by testing the growth on mineral medium agar plates as described for *Penicillium chrysogenum* in US 2002/0039758, without phenyl acetic acid but supplemented with 15 g/L agar to solidify and 0-50 mg/L phleomycin. No significant difference could be observed versus the WT. So, the *hdfB* deletion causes no apparent change towards phleomycin sensitivity. Finally, we concluded that, like the *hdfA* mutant, the *hdfB* mutant has no apparent phenotype and can be used for further studies.

### Example 3

### Homologous recombination frequencies in hdfA or hdfB mutant strains of Penicillium chrysogenum

To determine the homologous recombination frequency of *hdfA* and *hdfB* deletion strains compared to the WT strain, a deletion cassette for the *niaD* locus, encoding nitrate reductase, was constructed. To this end the two 1800 bp fragments of the *niaD* locus were amplified, these were separated by the *amdS* expression cassette and should enable the exchange of part of the *niaD* gene for the *amdS* expression cassette, thereby rendering the transformants chlorate resistant (due to the deletion of *niaD)* and able to use acetamide as the sole nitrogen source. Three PCR reactions were performed (see Table 2) and cloned into the Gateway pENTR vectors of Invitrogen, enabling later efficient fusion via the Multi-Gateway system (see www.lnvitrogen.com).

**Table 2. Oligonucleotides and pENTR vectors used for cloning of niaD-deletion cassette**

| Fragment | Template | Forward oligonucleotide | Reverse oligonucleotide | pENTR vector |
|---|---|---|---|---|
| 5'-niaD | Genomic DNA | SEQ ID NO 31 | SEQ ID NO 32 | pDONR™P4-P1R |
| PgpdA-amdS | pHELY-A1 | SEQ ID NO 33 | SEQ ID NO 34 | pDONR™221 |
| 3'-niaD | Genomic DNA | SEQ ID NO 35 | SEQ ID NO 36 | pDONR™P2R-P3 |

The three obtained vectors were used to perform a multi-site Gateway reaction into pDESTR4-R3, yielding plasmid pDESTR4R3NamdSN. This plasmid was linearized with EcoRI and transfected to *Penicillium chrysogenum* that were produced according to standard protocols (see for examples Cantoral et al., 1987, Biotechnology 5: 494-497; Swinkels et al., 1997, WO 97/06261) however Glucanex™ (Sigma) was applied as lysing enzyme. After transformation the protoplasts were plated out on selective regeneration agar plates with acetamide as the sole nitrogen source (for an exact description of the media, see Swinkels et al., 1997). Acetamide utilizing colonies were transferred to fresh acetamide plates (without saccharose to induce sporulation) and 100 of these were subsequently screened for chlorate sensitivity (per liter. NaCl, 3 g; KH₂PO₄, 1 g; FeSO₄. 7 H₂O, 10 mg; MgSO₄. 7 H₂O, 0.5 g; Oxoid Agar no.1, 15 g; Glucose, 20 g; spore elements, see WO9706261, page 10-11, all components from ZnSO₄ to biotin; adenine, 1.85 g; KClO₃, 12.5 g). From the results presented in Table 3 it can be clearly concluded that the deletion of either *hdfA* or *hdfB* has a significant effect on the frequency of targeted integration: from 1% in the WT to 47-56% in the mutants (i.e. the HR/NHR ratio improved from 0.01 in the parent eukaryotic cell to 0.47-0.56).

**Table 3. Gene targeting frequencies in WT, hdfA and hdfB mutants**

| Strain | Acetamide transformants | Chlorate resistant |
|---|---|---|
| WT | +/- 1400 | 1% |
| hdfA mutant SA1 | 222 | 56% |
| hdfB mutant SB 1 | 163 | 47% |

### Example 4

### Improved gene targeting frequencies in hdfA mutant strains of Penicillium chrysogenum using the bipartite gene-targeting method

In examples 1, 2 and 3 improved gene tartgeting frequencies were shown by using two methodologies known in the art. The double selection marker method of Liu et al (2001) in examples 1 and 2 show 16-63% correct gene targeting, while the deletion of either hdfA or hdfB (i.e. disturbing the NHEJ pathway) results in 47-56% correct gene targeting (see example 3). However, these frequencies are not efficient enough for high throughput gene targeting. To determine the homologous recombination frequency of *hdfA* deleted strains in combination with the bipartite method compared to the bipartite method alone various overlapping fragments were obtained via restriction digestion from plasmid pDESTR4R3NamdSN (Figure 5). Four different overlapping fragments were tested (see Figure 5 and Table 4). As controls the individual fragments were used or water. *Penicillium chrysogenum* protoplasts were produced according to standard protocols (see for examples Cantoral et al., 1987, Biotechnology 5: 494-497; Swinkels et al., 1997, WO97/06261) however Glucanex™ (Sigma) was applied as lysing enzyme. After transformation the protoplasts were plated out on selective regeneration agar plates with acetamide as the sole nitrogen source (for an exact description of the media, see Swinkels et al., 1997). Acetamide utilizing colonies were transferred to fresh acetamide plates (without saccharose to induce sporulation) and 100 of these were subsequently screened for chlorate sensitivity (per liter: NaCl, 3 g; KH₂PO₄, 1 g; FeSO₄. 7 H₂O, 10 mg; MgSO₄. 7 H₂O, 0.5 g; Oxoid Agar no.1, 15 g; Glucose, 20 g; spore elements, see WO9706261, page 10-11, all components from ZnSO₄ to biotin; adenine, 1.85 g; KClO₃, 12.5 g).

**Table 4. Overview and gene targeting frequencies in WT and ΔhdfA strains using bipartite gene-targeting method.**

| Strain | code | Overlap Fragment (kb) | Single Fragment | amdS transformants | Chlorate Resistant (%) |
|---|---|---|---|---|---|
| *ΔhdfA* | 5A | | | >300 | 0 |
| | 5B | 1.9 | | 177 | >95* |
| | 5C | 0.9 | | >500 | 100 |
| | 5D | 1.5 | | >500 | >98* |
| | 5E | 1.3 | | >500 | >98* |
| | | | | | |
| | | | H₂0 | 0 | - |
| | | | BamHl-BamHl | 1 | 0 |
| | | | Xbal-BstXl | 8 | 0 |
| | | | BamHl-Ndel | 0 | - |
| | | | Hpal-BstXl | 0 | - |
| WT | 5A | | | >750 | 1 |
| | 5B | 1.9 | | >2000 | 10 |
| | 5E | 1.3 | | >2000 | 10 |
| | | | | | |
| | | | H₂0 | 0 | - |
| | | | BamHl-BamHl | 9 | 0 |
| | | | Xbal-BstXl | >2000 | 0 |

| | | | | | |
|---|---|---|---|---|---|
| *: some amdS positive clones showed limited growth on the chlorate selection plates, but did show the right PCR pattern | | | | | |

From the results presented in Table 4 it is clear that the single Xbal-BstXl itself contains enough of the *amdS* open reading frame to convert the cells in acetamide utilizing cells. However, this single fragment with only one flanking sequence (i.e. the niaD 3'-flanking sequence) can only be integrated via a single or non-homologous recombination event and thus does not disturb the *niaD* locus (see Table 4: none of the tested *amdS*-positive WT transformants with the Xbal-Bstxl fragment is resistant versus chlorate). When this fragment is combined with the BamHl-BamHl fragment 10% of the amdS-positive transformants of the WT cells are resistant to chlorate, which is a significant increase as compared when using the same gene targeting cassette but than supplied as one fragment (see Table 4: sample 5A, resulting in 1% gene targeting; see also example 3). The same results are found when using a different overlap (the 1.3 kb overlap; Table 4: sample 5E). However, when the same method is applied in a *ΔhdfA* strain a surprising and remarkable difference was observed: using the individual fragments almost no transformants were obtained, in contrast to the overlapping fragments, suggesting an efficient recombination into functional selection markers. Moreover, at least 95% these colonies were shown to be chlorate resistant, indicative for proper gene replacement (and thus targeting) of the niaD locus. In fact, PCR reactions on sporulated colonies with oligonucleotides SEQ ID NO 37 and SEQ ID NO 11 confirmed that a 100% of the obtained transformants were correct gene targeting events. So, in conclusion by combining the single effects of improving the HR/NHR ratio ( 47-56% gene targeting; examples 1 and 2) and the bipartite gene-replacement method (10% gene targeting; example 4, Table 4, wild type results), we surprisingly find a 100% gene targeting. This means that the HR/NHR ratio of 0.01 of the parent eukaryotic cell was improved to 0.95-1 by combining methods that individually increased the HR/NHR ratio only to 0.47-0.56 and 0.1, respectively.

## Claims

1. A method to construct eukaryotic cells having a target sequence in a chromosomal DNA sequence replaced by a replacement sequence of interest comprising:
- modifying a parent eukaryotic cell with a preference for noun-homologous recombination to provide a eukaryotic cell having an increased homologous to non-homologous recombination ratio as compared to the parent cell,
- providing two sets of DNA molecules of which the first set comprises DNA molecules each comprising a first non-functional fragment of the replacement sequence of interest flanked at its 5'-side by a DNA sequence substantially homologous to a sequence of the chromosomal DNA flanking the target sequence and the second set comprises DNA molecules each comprising a second non-functional fragment of the DNA replacement sequence of interest overlapping with the first non-functional fragment and flanked at its 3'-side by a DNA sequence substantially homologous to a sequence of the chromosomal DNA flanking the target sequence, wherein the first and second non-functional fragments become functional upon recombination;
- transforming the modified eukaryotic cells with both sets of DNA molecules; and
- growing the cells to obtain transformed progeny cells having a DNA molecule from the first set recombined with a DNA molecule from the second set into a DNA molecule comprising a functional replacement sequence and having the target sequence replaced by the functional replacement sequence.

2. The method of claim 1, wherein modifying the parent eukaryotic cell is achieved by increasing the efficiency of the homologous recombination pathway and/or decreasing the efficiency of the non-homologous recombination pathway.

3. The method of claim 1 or 2, wherein modifying the parent eukaryotic cell is achieved by down regulating, preferably inactivating, equivalents of the yeast genes KU70, KU80, RAD50, MRE11, XRS2, LIG4, LIF1, NEJ1 and/or SIR4, involved in the non-homologous recombination pathway.

4. The method of any one of claims 1-3, wherein the two sets of DNA molecules are labeled with different fluorescent dyes for each set and the step of growing the cells to obtain transformed progeny cells comprises subjecting the cells to selection using fluorescence activated cell sorting.

5. The method of any one of claims 1-4, wherein the replacement sequence of interest comprises a selection marker, a modified version of the target sequence and/or additional copies of a sequence of interest being present in the genome of the eukaryotic cell.

6. The method of any one of claims 1-3, wherein the replacement sequence of interest comprises a selection marker and the step of growing the cells to obtain transformed progeny cells comprises selecting the cells by expression of the functional selection marker.

7. The method of claim 6, wherein the DNA molecules of the first set and/or the DNA molecules of the second set comprise an additional replacement sequence of interest.

8. The method of claim 6 or 7, wherein the selection marker is *amdS.*

9. The method of claim 1, wherein the DNA sequences flanking the functional replacement sequence have a degree of identity to a chromosomal DNA sequence flanking the target sequence of at least 80%.

## Patentansprüche

1. Verfahren zur Konstruktion eukaryotischer Zellen mit einer Zielsequenz in einer chromosomalen DNA-Sequenz, die durch eine Ersatzsequenz von Interesse ausgetauscht worden ist, umfassend:
- Modifizieren einer eukaryotischen Ausgangszelle mit einer Präferenz für nicht-homologe Rekombination zur Bereitstellung einer eukaryotischen Zelle mit einem erhöhten Verhältnis von homologer zu nicht-homologer Rekombination verglichen mit der Ausgangszelle,
- Bereitstellen von zwei Sätzen von DNA-Molekülen, wobei der erste Satz DNA-Moleküle umfasst, die jeweils ein erstes nicht-funktionelles Fragment der Ersatzsequenz von Interesse umfassen, das auf seiner 5'-Seite von einer DNA-Sequenz flankiert ist, die im Wesentlichen homolog zu einer Sequenz der chromosomalen DNA ist, die die Zielsequenz flankiert, und der zweite Satz DNA-Moleküle umfasst, die jeweils ein zweites nicht-funktionelles Fragment der DNA-Ersatzsequenz von Interesse umfassen, das mit dem ersten nichtfunktionellen Fragment überlappt und auf seiner 3'-Seite von einer DNA-Sequenz flankiert ist, die im Wesentlichen homolog zu einer Sequenz der chromosomalen DNA ist, die die Zielsequenz flankiert, wobei das erste und das zweite nicht-funktionelle Fragment nach der Rekombination funktionell werden,
- Transformieren der modifizierten eukaryotischen Zellen mit beiden Sätzen von DNA-Molekülen und
- Züchten der Zellen, um transformierte Nachkommenzellen zu erhalten, die ein DNA-Molekül aus dem ersten Satz, rekombiniert mit einem DNA-Molekül aus dem zweiten Satz zu einem DNA-Molekül umfasst, das eine funktionelle Ersatzsequenz umfasst, und wobei die Zielsequenz durch die funktionelle Ersatzsequenz ersetzt worden ist.

2. Verfahren nach Anspruch 1, wobei die Modifikation der eukaryotischen Ausgangszelle erzielt wird, indem man die Effizienz des homologen Rekombinationswegs erhöht und/oder die Effizienz des nicht-homologen Rekombinationswegs verringert.

3. Verfahren nach Anspruch 1 oder 2, wobei die Modifikation der eukaryotischen Ausgangszelle erzielt wird, indem man Äquivalente der Hefegene KU70, KU80, RAD50, MRE11, XRS2, LIG4, LIF1, NEJ1 und/oder SIR4, die am nicht-homologen Rekombinationsweg beteiligt sind, nach unten reguliert, vorzugsweise inaktiviert.

4. Verfahren nach einem der Ansprüche 1-3, wobei die zwei Sätze von DNA-Molekülen mit unterschiedlichen Fluoreszenzfarbstoffen für jeden Satz markiert werden und man bei dem Schritt des Züchtens der Zellen zum Erhalten transformierter Nachkommenzellen die Zellen einer Selektion unter Verwendung von fluoreszenzaktivierter Zellsortierung unterzieht.

5. Verfahren nach einem der Ansprüche 1-4, wobei die Ersatzsequenz von Interesse einen Selektionsmarker, eine modifizierte Version der zielsequenz und/oder zusätzliche Kopien einer Sequenz von Interesse, die im Genom der eukaryotischen Zelle vorhanden ist, umfasst.

6. Verfahren nach einem der Ansprüche 1-3, wobei die Ersatzsequenz von Interesse einen Selektionsmarker umfasst und man bei dem Schritt des Züchtens der Zellen zum Erhalten transformierter Nachkommenzellen die Zellen durch Expression des funktionellen Selektionsmarkers selektiert.

7. Verfahren nach Anspruch 6, wobei die DNA-Moleküle des ersten Satzes und/oder die DNA-Moleküle des zweiten Satzes eine zusätzliche Ersatzsequenz von Interesse umfassen.

8. Verfahren nach Anspruch 6 oder 7, wobei der Selektionsmarker *amdS* ist.

9. Verfahren nach Anspruch 1, wobei die DNA-Sequenzen, die die funktionelle Ersatzsequenz flankieren, einen Grad der Identität zu einer chromosomalen DNA-Sequenz, die die Zielsequenz flankiert, von mindestens 80% aufweisen.

## Revendications

1. Procédé pour construire des cellules eucaryotes ayant une séquence cible, dans une séquence d'ADN chromosomique, remplacée par une séquence de remplacement d'intérêt, comprenant les étapes consistant à :
- modifier une cellule eucaryote parent, avec une préférence pour une recombinaison non homologue, pour fournir une cellule eucaryote ayant un rapport accru d'une recombinaison homologue sur une non homologue, en comparaison avec la cellule parent,
- fournir deux ensembles de molécules d'ADN dont le premier ensemble comprend des molécules d'ADN comprenant chacune un premier fragment non fonctionnel de la séquence de remplacement d'intérêt flanqué, en son côté 5', d'une séquence d'ADN étant substantiellement homologue à une séquence de l'ADN chromosomique flanquant la séquence cible et le deuxième ensemble comprend des molécules d'ADN comprenant chacune un deuxième fragment non fonctionnel de la séquence de remplacement d'ADN d'intérêt qui se chevauche avec le premier fragment non fonctionnel et est flanqué, en son côté 3', d'une séquence d'ADN étant substantiellement homologue à une séquence de l'ADN chromosomique flanquant la séquence cible, dans lequel les premier et deuxième fragments non fonctionnels deviennent fonctionnels lors d'une recombinaison ;
- transformer les cellules eucaryotes modifiées avec les deux ensembles de molécules d'ADN ; et
- cultiver les cellules pour obtenir des cellules de la descendance transformées, ayant une molécule d'ADN provenant du premier ensemble recombinée avec une molécule d'ADN provenant du deuxième ensemble, dans une molécule d'ADN comprenant une séquence de remplacement fonctionnelle et ayant la séquence cible remplacée par la séquence de remplacement fonctionnelle.

2. Procédé selon la revendication 1, dans lequel la modification de la cellule eucaryote parent est accomplie en augmentant l'efficacité de la voie de recombinaison homologue et/ou en diminuant l'efficacité de la voie de recombinaison non homologue.

3. Procédé selon la revendication 1 ou la 2, dans lequel la modification de la cellule eucaryote parent est accomplie en régulant en baisse, de préférence en inactivant, des équivalents des gènes de levure KU70, KU80, RAD50, MRE11, XRS2, LIG4, LIF1, NEJ1 et/ou SIR4 impliqués dans la voie de recombinaison non homologue.

4. Procédé selon l'une quelconque des revendications 1 à 3, dans lequel les deux ensembles de molécules d'ADN sont marqués avec différents colorants fluorescents pour chacun des ensembles et l'étape de cultiver des cellules pour obtenir des cellules de la descendance transformées comprend la soumission des cellules à une sélection, en utilisant un tri de cellules activées par fluorescence.

5. Procédé selon l'une quelconque des revendications 1 à 4, dans lequel la séquence de remplacement d'intérêt comprend un marqueur de sélection, une version modifiée de la séquence cible et/ou des copies supplémentaires d'une séquence d'intérêt étant présentes dans le génome de la cellule eucaryote.

6. Procédé selon l'une quelconque des revendications 1 à 3, dans lequel la séquence de remplacement d'intérêt comprend un marqueur de sélection et l'étape de cultiver des cellules pour obtenir des cellules de la descendance transformées comprend la sélection des cellules via l'expression du marqueur de sélection fonctionnel.

7. Procédé selon la revendication 6, dans lequel les molécules d'ADN du premier ensemble et/ou les molécules d'ADN du deuxième ensemble comprennent une séquence de remplacement d'intérêt supplémentaire.

8. Procédé selon la revendication 6 ou la 7, dans lequel le marqueur de sélection est l*'amdS.*

9. Procédé selon la revendication 1, dans lequel les séquences d'ADN flanquant la séquence de remplacement fonctionnelle ont un degré d'identité, d'au moins 80%, avec une séquence d'ADN chromosomique flanquant la séquence cible.


### SEQUENCE LISTING

<110> DSM IP Assets B.V.
<120> IMPROVED METHOD FOR HOMOLOGOUS RECOMBINATION
<130> 26085WO
<150> EP07104588.4
   <151> 2007-03-21
<150> EP07111826.9
   <151> 2007-07-05
<160> 37
<170> PatentIn version 3.2
<210> 1
   <211> 33
   <212> DNA
   <213> Artificial
<220>
   <223> synthetic primer
<400> 1
   ttctctagaa acagctggca acgggcaatc tac 33
<210> 2
   <211> 46
   <212> DNA
   <213> Artificial
<220>
   <223> synthetic primer
<400> 2
   cgaggatacc tacttcgtaa caacattgat ttgaagtttg ctcagg 46
<210> 3
   <211> 45
   <212> DNA
   <213> Artificial
<220>
   <223> synthetic primer
<400> 3
   cctgagcaaa cttcaaatca atgttgttac gaagtaggta tcctc 45
<210> 4
   <211> 46
   <212> DNA
   <213> Artificial
<220>
   <223> synthetic primer
<400> 4
   cggatgcggc cgctgcgccc gggccctcga gacgattacg cattcc 46
<210> 5
   <211> 88
   <212> DNA
   <213> Artificial
<220>
   <223> synthetic primer
<400> 5
   ccagcggccg ccggcccggg cataacttcg tatagcatac attatacgaa gttatcctgc 60
   agggactctt tctggcatgc ggagagac 88
<210> 6
   <211> 43
   <212> DNA
   <213> Artificial
<220>
   <223> synthetic primer
<400> 6
   attgcggccg cggggcgcgc cgttgagtgg tatggggcca tcc 43
<210> 7
   <211> 85
   <212> DNA
   <213> Artificial
<220>
   <223> synthetic primer
<400> 7
   tcatggtacc agcccgggct ggcgcgccat aacttcgtat agcatacatt atacgaagtt 60
   atgttacgaa gtaggtatcc tcggg 85
<210> 8
   <211> 33
   <212> DNA
   <213> Artificial
<220>
   <223> synthetic primer
<400> 8
   ctttggtacc cttttcggcg agataatctc gac 33
<210> 9
   <211> 58
   <212> DNA
   <213> Artificial
<220>
   <223> synthetic primer
<400> 9
   gaacaaaagc ttggtggcgc gcccccggta ccggtcgact acatgtatct gcatgttg 58
<210> 10
   <211> 39
   <212> DNA
   <213> Artificial
<220>
   <223> synthetic primer
<400> 10
   gaaaaggaaa agcggccgca ttaaagcctt cgagcgtcc 39
<210> 11
   <211> 21
   <212> DNA
   <213> Artificial
<220>
   <223> synthetic primer
<400> 11
   gccacaggtg actctggatg g 21
<210> 12
   <211> 20
   <212> DNA
   <213> Artificial
<220>
   <223> synthetic primer
<400> 12
   taacagggag ataggtgaag 20
<210> 13
   <211> 21
   <212> DNA
   <213> Artificial
<220>
   <223> synthetic primer
<400> 13
   tgaagaagca cggctaattg g 21
<210> 14
   <211> 21
   <212> DNA
   <213> Artificial
<220>
   <223> synthetic primer
<400> 14
   gtctctccgc atgccagaaa g 21
<210> 15
   <211> 20
   <212> DNA
   <213> Artificial
<220>
   <223> synthetic primer
<400> 15
   atggttgaag atagctacac 20
<210> 16
   <211> 21
   <212> DNA
   <213> Artificial
<220>
   <223> synthetic primer
<400> 16
   aaacttctgc tcaaagtact g 21
<210> 17
   <211> 21
   <212> DNA
   <213> Artificial
<220>
   <223> synthetic primer
<400> 17
   ccaagacgat cctgagcaaa c 21
<210> 18
   <211> 20
   <212> DNA
   <213> Artificial
<220>
   <223> synthetic primer
<400> 18
   ggtcaatgac gctccattgg 20
<210> 19
   <211> 33
   <212> DNA
   <213> Artificial
<220>
   <223> synthetic primer
<400> 19
   caatctagac gacgatttga atgttttcca ccc 33
<210> 20
   <211> 47
   <212> DNA
   <213> Artificial
<220>
   <223> synthetic primer
<400> 20
   gccattttcc caacatcatc atcattggaa ttgagtgggg gggcggg 47
<210> 21
   <211> 47
   <212> DNA
   <213> Artificial
<220>
   <223> synthetic primer
<400> 21
   cccgcccccc cactcaattc caatgatgat gatgttggga aaatggg 47
<210> 22
   <211> 46
   <212> DNA
   <213> Artificial
<220>
   <223> synthetic primer
<400> 22
   ctggcggccg cttagcccgg gctcttgctt agcacggcac cgtacc 46
<210> 23
   <211> 87
   <212> DNA
   <213> Artificial
<220>
   <223> synthetic primer
<400> 23
   agacggtacc agcccgggca ggcgcgccat aacttcgtat agcatacatt atacgaagtt 60
   ataatgatga tgatgttggg aaaatgg 87
<210> 24
   <211> 31
   <212> DNA
   <213> Artificial
<220>
   <223> synthetic primer
<400> 24
   gcagggtacc gcgtcccaga ttgtcgggtc t 31
<210> 25
   <211> 20
   <212> DNA
   <213> Artificial
<220>
   <223> synthetic primer
<400> 25
   ccccttatga cccctctgtc 20
<210> 26
   <211> 23
   <212> DNA
   <213> Artificial
<220>
   <223> synthetic primer
<400> 26
   gcagatgatc gagtacatcc aag 23
<210> 27
   <211> 20
   <212> DNA
   <213> Artificial
<220>
   <223> synthetic primer
<400> 27
   atggcggaga aagaggctac 20
<210> 28
   <211> 19
   <212> DNA
   <213> Artificial
<220>
   <223> synthetic primer
<400> 28
   tgggagccag aaactatac 19
<210> 29
   <211> 22
   <212> DNA
   <213> Artificial
<220>
   <223> synthetic primer
<400> 29
   ttgaatagcg agacaataac cc 22
<210> 30
   <211> 21
   <212> DNA
   <213> Artificial
<220>
   <223> synthetic primer
<400> 30
   gcacaagggt tatatgcatc g 21
<210> 31
   <211> 63
   <212> DNA
   <213> Artificial
<220>
   <223> synthetic primer
<400> 31
   ggggacaact ttgtatagaa aagttggcgg ccgcggatcc gatcctcgat cttgcgatgt 60
   ttc 63
<210> 32
   <211> 49
   <212> DNA
   <213> Artificial
<220>
   <223> synthetic primer
<400> 32
   ggggactgct tttttgtaca aacttgccgt agaatccatt cggctatgg 49
<210> 33
   <211> 79
   <212> DNA
   <213> Artificial
<220>
   <223> synthetic primer
<400> 33
   ggggacaagt ttgtacaaaa aagcaggcta cgcgtaaggc gcgccaagcg gccgcgactc 60
   tttctggcat gcggagaac 79
<210> 34
   <211> 56
   <212> DNA
   <213> Artificial
<220>
   <223> synthetic primer
<400> 34
   ggggaccact ttgtacaaga aagctgggtc ccgggttgag tggtatgggg ccatcc 56
<210> 35
   <211> 50
   <212> DNA
   <213> Artificial
<220>
   <223> synthetic primer
<400> 35
   ggggacagct ttcttgtaca aagtgggagg tggccgaaga ataacaaaag 50
<210> 36
   <211> 67
   <212> DNA
   <213> Artificial
<220>
   <223> synthetic primer
<400> 36
   gggacaactt tgtataataa agttgggtac cgccagggaa ttcttgccac tccgtttatc 60
   catactg 67
<210> 37
   <211> 19
   <212> DNA
   <213> Artificial
<220>
   <223> synthetic primer
<400> 37
   ccattccgct ggattgagg 19